Europäisches Patentamt

(19) European Patent Office

Office européen des brevets

(11) Publication number: **0 230 762 B1**

# EUROPEAN PATENT SPECIFICATION

(12)

(45) Date of publication of patent specification :
24.04.91 Bulletin 91/17

(21) Application number : 86309920.6

(22) Date of filing : 18.12.86

(51) Int. Cl.⁵ : **G01N 33/52, C12Q 1/00,**
**G01N 33/68, G01N 27/26,**
**C12Q 1/50, C12Q 1/42,**
**C12Q 1/32**

(54) Use of dry analytical elements to determine electrically separated analytes.

(30) Priority : 19.12.85 US 811031

(43) Date of publication of application :
05.08.87 Bulletin 87/32

(45) Publication of the grant of the patent :
24.04.91 Bulletin 91/17

(84) Designated Contracting States :
CH DE FR GB IT LI SE

(56) References cited :
EP-A- 0 165 633
WO-A- 79/00044
FR-A- 2 328 194
US-A- 3 992 158
US-A- 4 246 084
US-A- 4 562 147
CHEMICAL ABSTRACTS, vol. 75, no. 1, 05 July
1971, Columbus, OH (US); J.DI GIORGIO:
"Determination of serum lactic dehydrogen-
ase iso-enzymes by use of the "Diagnostest"
cellulose acetate electrophoresis system", p.
262, no. 3114z
CHEMICAL ABSTRACTS, vol. 103, no. 23, 09
Dec 1985, Columbus, OH (US); E.HOLLER et
al.: "Non-disruptive detection of DNA
polymerases in nondenaturing polyac-
rylamide gels", p. 286, no. 191882s

(56) References cited :
CHEMICAL ABSTRACTS, vol. 103, no. 21, 25
Nov 1985, Columbus, OH (US); J.L.RIED et al.:
"Activity stain for rapid characterization of
pectic enzymes in isoelectric focusing and
sodium dodecyl sulfate-polyacrylamide gels",
p. 318, no. 174402t

(73) Proprietor : EASTMAN KODAK COMPANY (a
New Jersey corporation)
343 State Street
Rochester New York 14650 (US)

(72) Inventor : Emmons, Robert Edwin
Kodak Park
Rochester, NY 14650 (US)
Inventor : Mauck, Linda Ann
Kodak Park
Rochester, NY 14650 (US)
Inventor : Mauck, John Charles
Kodak Park
Rochester, NY 14650 (US)
Inventor : Wu, Tai-Wing, Dr., c/o Toronto
General Hospital
Eaton Wing 3-404B, 200 Elizabeth Street
Toronto, ON M5G 2C4 (CA)

(74) Representative : Nunney, Ronald Frederick
Adolphe et al
Kodak Limited Patent Department Headstone
Drive
Harrow Middlesex HA1 4TY (GB)

EP 0 230 762 B1

EP 0 230 762 B1

## Description

This invention relates to bioanalysis. In particular, it relates to a method for determining analytes which have been separated electrically, e.g. by electrophoresis. It also relates to a kit useful in this method.

A variety of analytical procedures have been developed for the separation and identification of different molecular species present in a specimen. Separation is generally accomplished by applying the specimen to a water-containing solid medium and inducing molecular separation of the species within the medium. In particular, chromatography and electrophoresis have been employed, both of which provide separation of different molecular species. The separation medium is generally called a chromatographic medium or electrophoretic plate. In such processes, a variety of reagents which interact with one or more of the molecular species in the specimen may also be applied to the medium before, during or after the separation process to assist in separation or identification of the analytes.

In general, poor resolution inhibits identification of separated materials in conventional electrophoretic media because of gross diffusion of the water-soluble reagents in the medium due to the presence of water. Proteins can be fixed to the medium with an acid or solvent to give quite sharp signals when stained. However, the fixing process usually destroys enzyme activity by denaturation. Further, the stains do not generally have the same affinity for all proteins to be determined.

Reagents have been introduced into the electrophoretic medium by a number of means. For example, it is known to use a transfer device composed of water-soluble reagents dispersed in a water-soluble binder to transfer reagents to an electrophoretic plate. When the transfer device is placed on the surface of an electrophoretic plate, the binder dissolves and the water-soluble reagents diffuse into the plate to react with the separated analytes. This technique has a number of disadvantages. The electrophoretic plate can be used only once because the reagents diffuse in water and the resulting detectable signal is observed in the plate. Moreover, because reagent transfer is carried out in a highly aqueous environment, resolution of the resulting analyte-reagent signals is poor. The reaction product (e.g. dyes) tends to spread out in the medium due to capillary action. Further, the procedure can be difficult to carry out.

It is also known to use a microporous nylon membrane to transfer analytes, e.g. nucleic acids, from an electrophoretic plate to the membrane by electroblotting. All analytes are transferred since the membrane has limited selectivity. The membrane is then treated to provide a detectable signal in the presence of analytes. It does not contain reagents which provide a detection means. Further, the transfer process is carried out in an aqueous environment.

Cellulosic membranes impregnated with water-soluble fluorogenic substrates have been described for use for identifying isoenzymes in an electrophoretic plate by R.E. Smith, in J. Histochem. Cytochem., 32 (12), pp. 1265-1274 (1984). This reference claims that the described membrane overlay provides highly resolved identification bands. However, these membranes have a number of serious drawbacks. The membrane overlay must be wet when used. As a result, the identification bands are quite diffuse because the substrates which provide a detectable signal are water-soluble. Further, the overlays do not provide a means for multiple testing with the same electrophoretic plate because substrate diffuses into the plate from the overlay. In essence, the Smith overlay can be used in a single test only.

In addition, the reference overlay membranes are not transparent and are therefore limited in utility. They cannot be used for transmission analysis using colorimetric detection procedures.

FR-A-2 328 194 also describes the use of a wet, substrate-containing cellulose acetate membrane as an overlay for measuring the concentration of electrophoretically separated isoenzymes.

The problems encountered with the known detection methods described above are overcome with a method for determining an analyte comprising the steps of :

A. forming a laminate by contacting a plate which is adapted for electrically induced migration and containing a plurality of analytes which have been separated from one another electrically, with an overlay and
B. removing the overlay from the plate and determining the analyte, said analyte being any substance which can be electrically induced to migrate through an aqueous medium and be separated from other substances on the basis of molecular charge,
the method characterized in that the overlay is a dry analytical element containing a water-insoluble binder material having dispersed therein an interactive composition which will react with at least one of the analytes to provide a non-diffusible, detectable species solely in the element, and wherein the detectable species is determined in the element.

Further, a method for determining an analyte in an aqueous liquid comprises the steps of :

A. contacting an electrophoretic plate with a sample of a liquid containing a plurality of analytes,
B. electrically separating the plurality of analytes from one another in the plate,
C. forming a laminate by contacting the plate with an overlay, and

2

D. removing the overlay from the plate and determining one of the analytes, said analyte being any substance which can be electrically induced to migrate through an aqueous medium and be separated from other substances on the basis of molecular charge,

the method characterized in that the overlay is a dry analytical element containing a water-insoluble binder material having dispersed therein an interactive composition which will react with at least one of the analytes to provide a non-diffusible, detectable species solely in the element, and wherein the detectable species is determined in the element.

A method for determining a plurality, (N), of analytes comprises the steps of :

A. forming a laminate by contacting a plate, which is adapted for electrically induced migration and containing a plurality of analytes which have been separated from one another electrically, with a first overlay, said analytes being any substances which can be electrically induced to migrate through an aqueous medium and be separated from other substances on the basis of molecular charge,

B. removing said first overlay from the plate,

the method characterized in that the first overlay is a dry analytical element containing a water-insoluble binder material having dispersed therein an interactive composition which will react with the first of the analytes to provide a first detectable species solely in that element, and wherein steps A and B are repeated up to N-1 times, using in each repetition, the plate and a respectively different dry analytical element containing a water-insoluble binder material having dispersed therein a respective interactive composition which will react with the respective analyte to provide a respective detectable species solely in the respectively different element,

provided that the interactive compositions of at least the first (N-1) determinations react with the respective analytes to provide respective non-diffusible detectable species in the respective elements.

A diagnostic kit of parts for the determination of one or more analytes comprises :

a plate adapted for electrically induced migration of an analyte, and

at least one dry analytical element comprising a water-insoluble binder material having dispersed therein an interactive composition which is capable of reacting with the analyte to provide a non-diffusible detectable species solely in the element.

The present invention provides a means to identify one or more analytes separated electrically using the same electrophoretic plate. In other words, this invention provides a multitest detection procedure. The method of this invention is easy to use, relatively inexpensive and highly sensitive. In preferred embodiments, it can be used with either colorimetric or fluorimetric detectable species.

These advantages are achieved by using as an overlay an analytical element containing a water-insoluble binder material having reagents dispersed therein which will react with the analyte of interest to provide a non-diffusible detectable species. Analyte reaction occurs at the interface of the plate and overlay to give very definitive bands. Since the detectable species is non-diffusible in water, it does not migrate from the overlay to the plate. Therefore, the same plate can be used repeatedly in an unlimited number of successive tests to determine one or more analytes.

Further, the assay is carried out under substantially dry conditions thereby minimizing the diffusion of the bands in the overlay, and the conditions encountered with known methods which reduce their sensitivity.

The present invention can be used to advantage in various fields of study including medicine, immunology, genetics, microbiology, biochemistry, clinical chemistry and forensic science to name a few. Any substance which can be electrically induced to migrate through an aqueous medium and be separated from other substances on the basis of molecular charge can be identified using this invention. Such substances are described herein as analytes. For example, this invention can be used for diagnostic purposes for detection of specific enzymes, antigens or antibodies in biological fluids, e.g. blood. In forensic science, it can be used in the phenotyping of genetic variants of enzymes and other proteins for the purpose of identification or individualization. Analyses of nucleic acids, peptides and other cellular components are also possible. In a preferred embodiment, the invention is useful for determinations of various proteins, such as enzymes (e.g. creatine kinase, lactate dehydrogenase, alkaline phosphate, glucose-6-phosphate dehydrogenase, adenylate kinase and γ-glutamyl transferase). The presence of isoenzymes that have been separated into discrete bands can be determined to advantage with this invention, including the presence of isoenzymes of creatine kinase, alkaline phosphatase and lactate dehydrogenase.

A plurality of analytes are generally present in an aqueous liquid, such as a human or animal biological fluid. The analytes can be separated by electrically inducing migration in a solid medium. A suitable sample of that liquid is applied to a suitable medium used in molecular migration or affinity separation procedures induced electrically. Such media include electrophoretic plates and electrofocusing plates. There are many commercially available plates which can be used in the method of this invention including those described, for example, in US-A-3 975 162 and those shown in the examples below. Generally, such plates have one or more compartments in a thin layer of a hydrated gel or membrane. Particularly useful are the plates prepared from hydrated gels, such as agarose, agar, polyacrylamide, acrylamide, and others known in the art. The agarose and polyacrylamide plates are preferred in the practice of this invention.

Once the liquid sample has been applied to the plate, it is subjected to a suitable electrical current to induce molecular migration and separation of the analytes, one from another. Known separation procedures can be used. For example, electrophoresis can be used whereby the analytes are subjected to an electrical field at constant pH and ionic strength. Another separation technique is electrofocusing whereby molecules are subjected to a pH gradient and separated on the basis of their individual isoelectric points.

After molecular separation has proceeded to a suitable extent, the surface of the electrophoretic plate is placed in contact with an analytical element (described below) in a manner that insures a minimum number of air bubbles at the interface. Generally, a laminate of the plate and element is formed by overlaying the plate with the element. However, the element can be overlayed with the plate. In any technique of contacting used in this invention, the surface of the element and the surface of the plate which contact form an interface at which reaction of analyte and interactive composition (described below) can occur. Since there is relatively little moisture present in this laminate, there is substantially no diffusion of analyte or non-diffusible detectable species across the interface. In other words, substantially no analyte leaves the plate and substantially no detectable species leaves the analytical element in the practice of this invention. This is attributable to the absence of a significant amount of water in either component of the plate-element laminate and the water-insolublity of the detectable species. There is some moisture in standard electrophoretic plates, but the amount is not sufficient to cause migration of water-insoluble reagents in the element.

The element and plate are kept together for a time sufficient to allow reaction of analyte and interactive composition to provide a detectable species solely in the element. The time for reaction will vary depending upon the analyte, the interactive composition used and the concentration of the analyte and composition. A minimal amount of routine experimentation may be needed to find the optimum time for reaction. The reaction is preferably carried out at 37°C in an incubation step, although any temperature up to 45°C can be used.

The detectable species produced as a result of the reaction will depend upon the type of interactive composition employed in the element. Generally, the species is detectable by spectrophotometric means. That is, the species can be measured with an increase or decrease in colorimetric, fluorometric, chemiluminescent or phosphorescent density which is detectable with suitable detection equipment and procedures. Preferably, the element is transparent so that the detectable chromogen is measured by transmission spectroscopy.

To determine the detectable species, the element is removed from the plate and subjected to appropriate detection procedures. If necessary, the element may be subjected to additional reagents or incubation to enhance the signal resulting from the species.

The analytical element used in the practice of this invention generally comprises one or more water-insoluble binder materials. The binder material and interactive composition dispersed therein are distributed throughout a zone which may be a coated self-supporting layer or a layer coated on a nonporous support. Elements of particular interest are those currently available from Eastman Kodak Co. (Rochester, New York, U.S.A.) as EKTACHEM® Clinical Chemistry analytical slides. Such elements can be modified by removal of the outermost porous spreading layers. Representative elements are illustrated in the Examples provided below. Other useful analytical elements are described, with or without spreading layers, in US-A-3 992 158, US-A-4 042 335, US-A-4 144 306, US-A-4 259 001 and US-A-4 430 436.

The elements generally comprise one or more zones or layers composed of one or more water-insoluble binder materials. It is essential that at least the outermost zone or layer of the element which will be contacted with the electrophoretic plate be composed of a water-insoluble binder material so that the element does not stick to the plate which may contain a minimal amount of water. Such a binder material is soluble in water to an extent of less than 1% by weight at 20°C. Useful binder materials include fibrous materials, such as filter paper, woven fabric, fibrous fleece or matting. In preferred embodiments, non-fibrous materials are used, such as natural or synthetic colloids (e.g. gelatin and polysaccharides which can be hardened to render them highly water-insoluble, acrylamide polymers, vinyl pyrrolidone polymers, cellulose esters, cellulose ethers, polyvinyl esters and vinyl acetals. Hardened gelatin is a preferred binder material.

The element can have one or more reagent layers containing one or more or all components of the interactive composition therein. Other layers can be present if desired, including subbing layers, radiation-blocking layers, reflective layers, registration layers, barrier layers, spreading layers, and the like as known by one skilled in the art. One layer may perform a multiplicity of functions, e.g. a spreading layer which is also a reagent layer. Preferably, the layers are carried on a suitable nonporous support which is usually a polymeric material, such as cellulose acetate, poly(ethylene terephthalate), a polycarbonate or a polyvinyl compound such as polystyrene. A support of choice will be compatible with the intended mode of detection. Preferred supports are radiation-transmissive which transmit electromagnetic radiation of a wavelength within the range between 200 and 900 nm as well as radiation due to radioactivity. Preferred supports are also relatively thin, e.g. less than 0.2 mm in thickness, in order to provide suitable flexibility for overlaying on an electrophoretic plate.

The interactive composition incorporated into the element will depend upon the analyte of interest. This invention is not intended to be limited in the scope of useful compositions which participate in one or more reactions to provide a non-diffusible detectable species in response to the analyte. A skilled worker in clinical chemistry would be able to determine a suitable composition for a particular analyte.

It is essential that the interactive composition provide non-diffusible species which can be detected in some manner in the element. The term "non-diffusible" means that the species is not able to diffuse out of the element nor move laterally within it to any appreciable extent. However, other components of the composition may be able to move within the element between zones or layers. Non-diffusibility is preferably attained with water-insoluble species. These water-insoluble species may be coated in organic solvents which solubilize the species for coating or immobilizing purposes. Examples of useful non-diffusible species are dyes formed from water-insoluble leuco dyes, e.g. the triarylimidazoles describes in US-A-4 089 747. Other useful non-diffusible species or their precursors are known to those having ordinary skill in the art.

Alternatively, the detectable species can be water-soluble, but rendered non-diffusible in the element in a suitable fashion, such as with mordants or immobilizing binders. Such water-soluble species and immobilizing materials are numerous and known to one of ordinary skill in the art.

In a preferred embodiment of this invention, an analytical element designed to determine creatine kinase (CK) is used to separate and identify CK isoenzymes. One such element is described in Example 1 below. The interactive composition in that element includes peroxidase, α-glycerophosphate oxidase, a water-insoluble triarylimidazole leuco dye which is capable of providing a water-insoluble detectable dye in response to the reaction of the isoenzymes, adenosine diphosphate, glycerol and glycerol kinase. These reagents can be located in the same or different layers of the element. Other creatine kinase interactive compositions are known in the art.

A significant advantage of the method of this invention is that it can be used to determine a plurality (identified as N herein) of analytes successively using a single electrophoretic plate and liquid sample and a multiplicity of dry analytical elements.

The detectable species produced in each element in this embodiment can be measured immediately after the element is removed from the plate, or the element can be kept in an appropriate environment for measurement later. The measurement of a plurality of analytes according to this embodiment is illustrated in Example 3 below.

Alternatively, in a series of determinations, the last determination can be made with a diffusible detectable species as long as the preceding determinations are made with a non-diffusible species. Because the detectable species produced in the last test is diffusible, it may migrate into the plate thereby contaminating it for further tests.

In yet another embodiment, the last two determinations of a series of determination can be made with different diffusible interactive compositions in succession if the detectable species produced thereby are distinct and can be separately measured. For example, one test can produce a fluorometric signal while the second test can produce a colorimetric signal. These determinations, however, must follow one or more tests wherein non-diffusible interactive species are produced and measured.

The analytical element and a plate (electrophoretic or electrofocusing) adapted for electrically induced migration of an analyte can be obtained individually, or together as a diagnostic kit. More than one element may be included in the kit either for the same analyte or for different analytes.

The examples below illustrate the practice of the invention.

As used in the context of this disclosure, I.U. represents the International Unit for enzyme activity defined as one I.U. being the amount of enzyme activity required to catalyze the conversion of 1 micromole of substrate per minute under standard pH and temperature conditions for the enzyme.

## Methods Used in the Examples

A. Electrophoretic methods of the art were performed according to procedures recommended by the manufacturers of the equipment used. Samples were spiked with 20 mmole of N-acetyl-L-cystein to enhance creatine kinase (CK) performance. N-acetyl-L-cystein was not present in the analytical element. Highlights of these methods are :

1. CK Electrophoresis Procedure, Helena Laboratories, Procedure No. 20, (dated 8/80). The isoenzymes of CK are separated according to their electrophoretic mobility on a cellulose acetate electrophoretic plate in a tris-barbital buffer. After separation, the cellulose acetate plates are laminated to a Helena CK Isoenzyme Reagent substrate overlay and incubated at 37-40°C.

At the end of the incubation period (25-30 minutes), the laminated materials were separated and the stained cellulose acetate plates were completely dried in an incubator.

The resulting CK bands on the stained cellulose acetate plate were visualized using a UV lamp or quantitated in a densitometer using either visible light or ultraviolet light and fluorescence quenching.

2. Analytical Electrofocusing in Polyacrylamide Gel (PAG) :

| Ampholyte PAG Plates | Compositions pH 3.5–9.5 | pH 5.5–8.5 |
|---|---|---|
| Anode Electrode Solution buffer | 1 Molar $H_3PO_4$ | 0.4 Molar HEPES** buffer |
| Cathode Electrode Solution | 1 Molar NaOH | 0.1 Molar NaOH |
| Cooling | 10°C | 10°C |
| Power Supply Settings | Power = 30 Watts Voltage = 1800 Current = Maximum | Power = 30 Watts Voltage = 1800 Current = Maximum |
| Time | 1.5 Hours | 2.5 Hours |

3. Analytical Electrofocusing (EF) in Agarose :

| Analytical EF in Agarose | Compositions pH 3.5–9.5 | pH 5.0–8.0 |
|---|---|---|
| Anode Electrode Solution | 0.5 Molar Acetic Acid | 0.04 Molar L– Glutamic Acid |
| Cathode Electrode Solution | 0.5 Molar NaOH | 0.5 Molar NaOH |
| Cooling | 10°C | 10°C |
| Power Supply Settings | Voltage = Maximum Current = Maximum Power = Initially zero, then in– crease until 500 V is attained* | Voltage = Maximum Current = Maximum Power = Initially zero, then in– crease until 500 V is attained* |
| Time | 30 Minutes | 30 Minutes |

*Setting electrical parameters in this manner enables one to use the recommended settings for any sized gel.

** HEPES buffer is 4–(2–hydroxymethyl)–1–piperazineethanesulfonic acid

B. Electrofocusing was carried out in the following manner.

1. The cooling plate of a standard Multiphor electrophoresis apparatus was coated with kerosene.

2. The template was laid on the cooling plate and any air bubbles were removed.

3. The template was coated with kerosene.

4. The agarose gel was laid on top of the template with the plastic side downward to avoid trapping air bubbles.

5. The electrode strips were evenly saturated with the appropriate electrode solutions. A faint trail of moisture remained on the foil if the strips were properly wetted. Any excess solution was removed by blotting.

6. The wicks were laid, as straight as possible, on the proper position as indicated by the template.

7. The wicks were cut sharply just short of the edge of the gel.

8. The samples were applied as desired, avoiding the edges of the gel. The sample volume was 15-20 $\mu\ell$. Small volumes (e.g. 2 $\mu\ell$) were directly pipetted onto the gel surface. For most proteins, the best application site is 2-4 cm from the cathode strip. High-molecular-weight proteins were applied close to their expected pI positions.

9. The electrofocusing lid for focusing across the gel was applied, the electrode wires were aligned along the electrode strips, and the red and black wires were connected.

10. The pressure bar (not needed for gels with pH 3.5-9.5 range ampholytes) was inserted into the safety lid, end the lid was placed in position.

11. The power supply was turned on.

12. After 20-30 minutes of focusing, the power was turned off and the sample application pieces were removed. The lids were replaced and focusing continued at the same power settings.

Example 1 : Determination of Creatine Kinase (CK) Isoenxymes – A Comparison of Present Invention with Known Method

A repeating series of serum samples was applied to a polyacrylamide gel plate containing ampholytes in the pH range of 3.5-9.5. The samples were electrofocused for 90 minutes according to the procedure described above. Following this, the plate was cut in half and excess moisture removed. To one half, a modified EKTACHEM® dry analytical element (described below) for the detection (visualization) of CK isoenzymes was applied according to the present invention. To the other half, a warm substrate-dye, agarose solution was overlayed and allowed to cool to form a Control electrophoretic plate-overlay laminate. Both halves were placed in an incubator and allowed to react for the same length of time at 37°C.

Both overlays were removed from the respective PAG plates. Upon observation, the bands on the dry analytical element used in this invention were clearly defined whereas those on the agarose overlay (Control) were diffuse and poorly defined. Moreover, bands were evident on the Control electrophoretic plate indicating that diffusion occurs from the overlay to the plate. No bands were evident on the plate used according to this invention.

The analytical element used for the determination of electrophoretically separated creatine kinase isoenzymes described above had the following format :

| | |
|---|---|
| Gelatin (hardened) | 5 g/m$^2$ |
| TRITON®X-200E surfactant | 0.1 g/m$^2$ |
| Bis Tris Buffer* | 2 g/m$^2$ |
| Creatine Phosphate | 1.7 g/m$^2$ |
| Adenosine Diphosphate (ADP) | 0.2 g/m$^2$ |
| Adenosine Monophosphate (AMP) | 0.2 g/m$^2$ |
| Glycerol | 0.2 g/m$^2$ |
| Magnesium Acetate | 0.2 g/m$^2$ |
| Diadenosine Pentaphos- phate (DAPP) | 0.005 g/m$^2$ |
| Glycerol Kinase | 4500 I.U./m$^2$ |

| | |
|---|---|
| Gelatin | 5 g/m$^2$ |
| Tris Buffer ** | 2 g/m$^2$ |
| Dimedone | 0.05 g/m$^2$ |
| 2-(3,5-dimethoxy-4-hydroxy- phenyl)-4,5-bis(4-di- methylaminophenyl)- imidazole | 0.2 g/m$^2$ |
| 2,4-Di-n-pentylphenol | 1.3 g/m$^2$ |
| ALKANOL®XC surfactant | 0.3 g/m$^2$ |
| TRITON®X-200E surfactant | 0.1 g/m$^2$ |
| Glycolic Acid | 0.3 g/m$^2$ |
| Ascorbic acid oxidase | 11,000 I.U./m$^2$ |
| Peroxidase | 33,000 I.U./m$^2$ |
| α-Glycerophosphate oxidase | 3,200 I.U./m$^2$ |

Support ///////////////////////////

*Bis Tris = 2-bis(2-hydroxyethyl)amino-2-(hydroxymethyl)-1,3-propanediol

** Tris = tris(hydroxymethyl)amino-methane

Example 2 : Creatine Kinase Isoenzyme Determination – Comparison of Method of Present Invention with Cellulose Acetate Electrophoresis Plate Method of Helena Laboratories

Example 1 was repeated using the CK electrophoresis procedure of Helena Laboratories and the method of the present invention.

Two plates containing cellulose acetate medium were soaked in electrode buffer, blotted and identical serum samples applied to each. Both plates were placed in a standard Helena chamber prepared as outlined in the manufacturer's instructions. Following electrophoresis, the plates were removed and treated as follows:

1. One plate was laminated with a dry analytical element as described in Example 1 above. Bubbles were removed at the interface, and the laminate was incubated at 37°C.

2. The other plate was laminated with a second cellulose acetate plate that had been prepared by :

    a. Being soaked in electrode buffer and then blotted.

    b. Loaded with the substrate-dye solution by coating the cellulose acetate with the CK reagent and allowing it to absorb.

The resulting laminate was placed in the incubator at 37°C to develop. At the end of a specified period of time, both laminates were removed from the incubator and the overlays stripped off the plates.

The bands on both the cellulose acetate overlay and its corresponding electrophoresis plate were not visible under normal room lighting. When dried and placed under ultra-violet light, both the overlay and the electrophoresis plate showed bands. The bands on the overlay were very weak compared to those on the plate. The overlay bands were also diffuse.

In contrast, the test according to this invention using the dry analytical element showed bands in normal room light. The corresponding electrophoresis plate showed no bands. When the element and the corresponding cellulose acetate electrophoresis plate were placed under ultra-violet light, the element showed bands that were quenched against the fluorescent background. No bands were seen on the cellulose acetate electrophoresis plate because reagents and reaction products did not diffuse into the electrophoresis plate from the element. The bands present on the element were broader than those seen after isoelectric focusing. This is because without the focusing effect of electrophoresis in a pH gradient, the CK isoenzymes tend to diffuse during separation in an electric field. Despite this, the dry element was still much sharper than the plate using a cellulose acetate overlay.

Example 3 : <u>Multiple Analysis Using a Single Electrophoretic Plate</u>

The CK element used was that described in Example 1.

The alkaline phosphatase (ALP) element used in this assay is shown below. Titanium dioxide was placed in the gel layer to provide a white background for the yellow dye generated during isoenzyme band development.

| | Gelatin (hardened) | 22 $g/m^2$ |
| --- | --- | --- |
| | Mg Chloride | 0.02 $g/m^2$ |
| | p-Nitrophenyl Phosphate | 0.54 $g/m^2$ |
| | TRITON® X-102 surfactant | 1.3 $g/m^2$ |
| Reagent | Tris(hydroxymethyl)amino- | 3 $g/m^2$ |
| Layer | methane buffer (pH 8) | |
| | Poly(styrene-co-N-vinyl- | 3 $g/m^2$ |
| | benzyl-N,N-dimethylbenzyl- | |
| | ammonium chloride-co-di- | |
| | vinylbenzene) | |
| | | |
| Reflective | $TiO_2$ | 30 $g/m^2$ |
| Gel Layer | Gelatin (hardened) | 22 $g/m^2$ |
| | Tris(hydroxymethyl)amino- | 3 $g/m^2$ |
| | methane buffer (pH 8) | |
| | | |
| Support | / / / / / / / / / / / / / / / / / / / / | |

Serum samples were applied to two polyacrylamide gel plates and electrophoresis separation was performed as described above.

To plate I, the ALP element was applied and incubated as described above for 3-5 minutes. This element overlay developed yellow bands characteristic of ALP reaction using this substrate. No bands developed in the gel plate because no appreciable dye diffusion occurred during the short time of reaction.

The second plate II was overlayed with the modified CK element described in Example 1. Similarly to Example 1, clear bands were evident in the overlay. No bands were developed in the gel plate.

A fresh set of elements were again overlayed on the same polyacrylamide gel plates. This time, however, the order of overlay was reversed. The CK element was applied to plate I and the ALP element to plate II. The resulting laminates were incubated and dried as described above.

Both elements developed bands characteristic in color and appearance to the analytes they were designed to detect. The electrophoretic gels showed no band formation in either case.

This example demonstrates the multitest capability of the present invention. Successive application of dry analytical elements to a single electrophoretic plate can be used to determine those analytes for which the elements are designed to detect.

Example 4 : Comparison of Present Invention with Method of the Prior Art

This example compares the method of the present invention to an analyte determination using an "Enzyme Overlay Membrane" described by R. E. Smith in J. Histochem. Cytochem. 32 (12), pp. 1265-1274 (1984).

Enzyme overlay membranes (EOMs) comprising substrate-impregnated cellulose acetate for the detection of trypsin isoenzymes, were purchased from Enzyme Systems Products (Livermore, California, U.S.A.). These membranes contain a fluorogenic trypsin substrate to detect isoenzyme activity pat-terns on agarose and polyacrylamide electrophoresis plates.

Isoelectric focusing was performed on 0.5 mm thick polyacrylamide gel plates using a LKB horizonlal electrophoresis system at a pH range of 3.5-9.5. The electrofocusing temperature was maintained at 5°C. The power supply settings were : voltage = 1800, power = 20 watts, current = 200 milliamps. Focusing was carried out for about 2.5 hours.

Patient serum samples suspected of having elevated trypsin activity were prepared as follows :

(1) left in native state, (2) spiked with bicarbonate-buffered trypsin (pH 6.6) having a calculated activity in excess of 1000 I.U./1, (3) a trypsin-spiked sample diluted to 1 : 1 with buffer, (4) a trypsin-spiked sample diluted 3 : 1 with buffer.

Each serum sample was subjected to electrophoresis as described above. An EOM was dipped into a bath of distilled water to moisten it, and was carefully laid over each gel plate so that no air bubbles were entrapped. The resulting laminates were incubated for 10-30 minutes at 37°C. An ultraviolet (long-wave) lamp was used to monitor the reactions in each laminate. When optimal fluorescence was obtained, the overlays were peeled off while the gel plates were still moist. The membranes were allowed to dry. Observation of the membranes and gel plates revealed the following :

The first serum sample showed slight activity in both application areas of the plate, i.e. at pH 8.5 and at pH 4.0. A very light streak of diffuse fluorescence connected the areas of primary activity.

The trypsin-spiked sample showed primary activity in the same regions. However, activity in the region between the primary bands resolved into more well defined bands surrounded by diffuse fluorescence. Dilutions of the spiked sample showed decreasing fluorescence that approximately correlated with the expected decreasing activity. The fluorescence appears to be unaffected by the pH gradient of the isoelectric focusing gel in the trypsin assay evaluated.

In order to check for diffusion of fluorescent substrate into or onto the gel plates, a sheet of wet, untreated cellulose acetate was applied to a gel plate previously overlayed with an EOM. Observation of the dried cellulose acetate showed diffuse fluorescence in the areas of EOM-gel reactions.

The disadvantages of using the commercially available EOMs are evident : (1) the EOM must be wet to activate the substrate, causing unwanted diffusion of the soluble substrates, (2) upon multiple testing, subsequent overlays exhibited progressively less distinct bands, (3) substrates useful in routine clinical analyses of serum components are not available (4) the EOMs are not transparent and therefore cannot be used for direct or transmission spectrophotometry, and (5) they are relatively expensive.

The assay described above was compared to an assay carried out according to the present invention as described in Example 1 above. The present invention provided highly distinct bands in the dry element whereas the electrophoretic plate had no observable bands and could be reused.

## Claims

1. A method for determining an analyte comprising the steps of :

A. forming a laminate by contacting a plate which is adapted for electrically induced migration and containing a plurality of analytes which have been separated from one another electrically, with an overlay and

B. removing the overlay from the plate and determining the analyte, said analyte being any substance which can be electrically induced to migrate through an aqueous medium and be separated from other substances on the basis of molecular charge,

the method characterized in that the overlay is a dry analytical element containing a water-insoluble binder material having dispersed therein an interactive composition which will react with at least one of the analytes to provide a non-diffusible, detectable species solely in the element, and wherein the detectable species is determined in the element.

2. A method for determining an analyte in an aqueous liquid comprising the steps of :

A. contacting an electrophoretic plate with a sample of a liquid containing a plurality of analytes,

B. electrically separating the plurality of analytes from one another in the plate,

C. forming a laminate by contacting the plate with an overlay, and

D. removing the overlay from the plate and determining one of the analytes, said analyte being any substance which can be electrically induced to migrate through an aqueous medium and be separated from other substances on the basis of molecular charge,

the method characterized in that the overlay is a dry analytical element containing a water-insoluble binder material having dispersed therein an interactive composition which will react with at least one of the analytes to provide a non-diffusible, detectable species solely in the element, and wherein the detectable species is determined in the element.

3. A method for determining a plurality, (N), of analytes comprising the steps of :

A. forming a laminate by contacting a plate, which is adapted for electrically induced migration and containing a plurality of analytes which have been separated from one another electrically, with a first overlay, said analytes being any substances which can be electrically induced to migrate through an aqueous medium and be separated from other substances on the basis of molecular charge,

B. removing said first overlay from the plate,

the method characterized in that the first overlay is a dry analytical element containing a water-insoluble binder material having dispersed therein an interactive composition which will react with the first of the analytes to provide a first detectable species solely in that element, and wherein steps A and B are repeated up to N-1 times, using in each repetition, the plate and a respectively different dry analytical element containing a water-insoluble binder material having dispersed therein a respective interactive composition which will react with the respective analyte to provide a respective detectable species solely in the respectively different element,

provided that the interactive compositions of at least the first (N-1) determinations react with the respective analytes to provide respective non-diffusible detectable species in the respective elements.

4. The method as claimed in any of claims 1 to 3 wherein after each step of contacting the plate with the overlay and before each step of removing the overlay from the plate, the respective plate-element laminate is incubated at a temperature of up to 45°C and for a time sufficient for the respective analyte to react with the respective interactive composition.

5. A diagnostic kit of parts for the determination of one or more analytes comprising

a plate adapted for electrically induced migration of an analyte, and

at least one dry analytical element comprising a water-insoluble binder material having dispersed therein an interactive composition which is capable of reacting with the analyte to provide a non-diffusible detectable species solely in the element.

6. The invention as claimed in any of claims 1 to 5 wherein the plurality of analytes are proteins or nucleic acids.

7. The invention as claimed in any of claims 1 to 6 wherein the plurality of analytes are isoenzymes.

8. The invention as claimed in any of claims 1 to 7 wherein the plurality of analytes are isoenzymes of creatine kinase, alkaline phosphatase or lactate dehydrogenase.

9. The invention as claimed in any of claims 1 to 8 wherein each analytical element is essentially transparent and each interactive composition reacts to provide a non-diffusible chromogen.

10. The invention as claimed in any of claims 1 to 9 wherein each dry analytical element comprises a support having thereon at least one layer containing the binder material and the interactive composition.

11. The invention as claimed in any of claims 1 to 10 wherein the element binder material is hardened gelatin.

## Ansprüche

1. Verfahren zur Bestimmung eines Analyten mit den Stufen :

A. Bildung eines Laminates durch Inkontaktbringen einer Platte, die für eine elektrisch induzierte Wanderung geeignet ist und eine Mehrzahl von Analyten enthält, die auf elektrischem Wege voneinander getrennt worden sind, mit einem Überzug und

B. Entfernung des Überzuges von der Platte und Bestimmung des Analyten, wobei der Analyt eine beliebige Substanz ist, die, wenn sie elektrisch induziert wird, durch ein wäßriges Medium wandert und von anderen Substanzen aufgrund der molekularen Ladung getrennt wird, dadurch gekennzeichnet, daß der Überzug ein trockenes analytisches Element ist, das ein in Wasser unlösliches Bindemittel enthält, in dem eine reaktive Zusammensetzung dispergiert ist, die mit mindestens einem der Analyten unter Bildung eines nicht-diffundierenden, nur im Element bestimmbaren Stoffes reagiert, und bei dem der bestimmbare Stoff im Element bestimmt wird.

2. Verfahren zur Bestimmung eines Analyten in einer wäßrigen Flüssigkeit mit den Stufen :

A. Inkontaktbringen einer elektrophoretischen Platte mit einer Probe einer Flüssigkeit, die eine Mehrzahl von Analyten enthält,

B. elektrische Trennung der Mehrzahl von Analyten voneinander in der Platte,

C. Bildung eines Laminates durch Inkontaktbringen der Platte mit einem Überzug, und

D. Entfernung des Überzuges von der Platte und Bestimmung eines Analyten, wobei der Analyt eine beliebige Substanz ist, die, wenn sie elektrisch induziert wird, durch ein wäßriges Medium zu wandern vermag und von anderen Substanzen aufgrund der molekularen Ladung getrennt wird, dadurch gekennzeichnet, daß der Überzug ein trockenes analytisches Element ist, das ein in Wasser unlösliches Bindemittel enthält, in dem eine reaktive Zusammensetzung dispergiert ist, die mit mindestens einem der Analyten unter Bildung eines nicht-diffundierenden, nur im Element bestimmbaren Stoffes reagiert, und bei dem der bestimmbare Stoff im Element bestimmt wird.

3. Verfahren zur Bestimmung einer Mehrzahl (N) von Analyten mit den Stufen :

A. Bildung eines Laminates durch Inkontaktbringen einer Platte, die für eine elektrisch induzierte Wanderung geeignet ist und eine Mehrzahl von Analyten, die auf elektrischem Wege voneinander getrennt worden sind, enthält, mit einem ersten Überzug, wobei die Analyten beliebige Substanzen sind, die, wenn sie elektrisch induziert werden, durch ein wäßriges Medium zu wandern vermögen, und von anderen Substanzen aufgrund der molekularen Ladung getrennt werden,

B. Entfernung des ersten Überzuges von der Platte, dadurch gekennzeichnet, daß der erste Überzug ein trockenes analytisches Element ist, das ein in Wasser unlösliches Bindemittel enthält, in dem eine reaktive Zusammensetzung dispergiert ist, die mit dem ersten der Analyten unter Bildung eines nur im Element bestimmbaren Stoffes reagiert, und bei dem die Stufen A und B bis zu N-1 Male wiederholt werden, wobei bei jeder Wiederholung die Platte und ein entsprechend unterschiedliches trockenes analytisches Element mit einem in Wasser unlöslichen Bindemittel, in dem eine entsprechende reaktive Zusammensetzung dispergiert ist, die mit dem entsprechenden Analyten unter Bildung eines entsprechenden bestimmbaren Stoffes allein in dem entsprechenden unterschiedlichen Element reagiert, verwendet werden,

wobei gilt, daß die reaktiven Zusammensetzungen mindestens der ersten (N-1) Bestimmungen mit den entsprechenden Analyten reagieren, unter Bildung entsprechender nichtdiffundierender bestimmbarer Stoffe in den entsprechenden Elementen.

4. Verfahren nach einem der Ansprüche 1 bis 3, bei dem nach jeder Stufe des Inkontaktbringens der Platte mit dem Überzug und vor jeder Stufe der Entfernung des Überzuges von der Platte, das entsprechende Platten-Element-Laminat bei einer Temperatur von bis zu 45°C und eine solche Zeitspanne lang inkubiert wird, die ausreicht, damit der entsprechende Analyt mit der entsprechenden reaktiven Zusammensetzung reagiert.

5. Diagnostische Packung von Teilen für die Bestimmung von einem oder mehreren Analyten mit :

einer Platte, die für eine auf elektrischem Wege induzierte Wanderung eines Analyten geeignet ist, und mindestens einem trockenen analytischen Element mit einem in Wasser unlöslichen Bindemittel, in dem eine reaktive Zusammensetzung dispergiert ist, die mit einem Analyten unter Bildung eines nicht-diffundierenden bestimmbaren Stoffes nur im Element zu reagieren vermag.

6. Die Erfindung nach einem der Ansprüche 1 bis 5, in der die Mehrzahl von Analyten Proteine oder Nukleinsäuren sind.

7. Die Erfindung nach einem der Ansprüche 1 bis 6, in der die Vielzahl von Analyten Isoenzyme sind.

8. Die Erfindung nach einem der Ansprüche 1 bis 7, in der die Vielzahl von Analyten Isoenzyme von Kreatinkinase, alkalischer Phosphatase oder Lactatdehydrogenase sind.

9. Die Erfindung nach einem der Ansprüche 1 bis 8, in der jedes analytische Element praktisch transparent ist und jede reaktive Zusammensetzung unter Bildung eines nichtdiffundierenen Chromogens reagiert.

10. Die Erfindung nach einem der Ansprüche 1 bis 9, in der jedes trockene analytische Element einen Träger aufweist, auf dem sich mindestens eine Schicht befindet, die das Bindemittel und die reaktive Zusammensetzung enthält.

11. Die Erfindung nach einem der Ansprüche 1 bis 10, in der das Bindemittel des Elementes gehärtete Gelatine ist.

## Revendications

1. Méthode pour la détermination d'une substance à analyser, comprenant les étapes suivantes :

A. on forme un laminé en mettant en contact une plaque qui est adaptée à une migration électro-induite et contient plusieurs substances à analyser qui ont été séparées les unes des autres électriquement, avec une surcouche et

B. on enlève la surcouche de la plaque et on détermine la substance à analyser, ladite substance étant capable d'une migration électro-induite à travers un milieu aqueux et étant séparable des autres substances en fonction de la charge des molécules,

cette méthode étant caractérisé en ce que la surcouche est un élément d'analyse à sec contenant un liant insoluble dans l'eau, avec, dispersé dans ce liant, une composition interactive qui réagit avec au moins l'une des substances à analyser pour former seulement dans l'élément une espèce non-diffusible et détectable, cette espèce étant déterminée dans l'élément.

2. Méthode pour déterminer une substance à analyser dans un liquide aqueux, comprenant les étapes suivantes :

A. on met en contact une plaque d'électrophorèse avec un échantillon de liquide contenant plusieurs substances à analyser,

B. on sépare électriquement les substances à analyser les unes des autres,

C. on forme un laminé en mettant en contact la plaque et une surcouche et

D. on enlève la surcouche de la plaque et on détermine l'une des substances à analyser, ladite substance à analyser étant capable d'une migration électro-induite à travers un milieu aqueux, et séparable des autres substances en fonction de la charge des molécules,

cette méthode étant caractérisé en ce que la surcouche est un élément d'analyse à sec contenant un liant insoluble dans l'eau, avec, dispersé dans ce liant, une composition interactive qui réagit avec au moins l'une des substances à analyser pour former seulement dans l'élément une espèce non-diffusible détectable, cette espèce étant déterminée dans l'élément.

3. Méthode pour déterminer plusieurs substances à analyser, comprenant les étapes suivantes :

A. on forme un laminé en mettant en contact une plaque qui est adaptée à une migration électro-induite et qui contient plusieurs substances à analyser qui ont été séparées les unes des autres électriquement, avec une première surcouche, les substances à analyser étant capables d'une migration électro-induite à travers un milieu aqueux et séparable des autres substances en fonction de la charge des molécules,

B. on enlève cette première surcouche de la plaque, cette méthode étant caractérisé en ce que la première surcouche est un élément d'analyse à sec contenant un liant insoluble dans l'eau, avec, dispersé dans ce liant, une composition interactive qui réagit avec au moins l'une des substances à analyser pour former une espèce détectable seulement dans l'élément, et en ce que les étapes A et B sont répétées jusqu'à N-1 fois, en utilisant à chaque répétition la plaque et un élément d'analyse à sec respectivement différent et contenant un liant insoluble dans l'eau dans lequel est dispersée une composition interactive respective qui réagit avec la substance à analyser respective pour former une espèce détectable respective seulement dans l'élément différent respectif, avec la condition que les compositions interactives d'au moins les premières (N-1) déterminations réagissent avec les substances à analyser respectives pour fournir des espèces détectables non-diffusibles dans les éléments respectifs.

4. Méthode selon l'une des revendications 1 à 3, où après chaque étape de mise en contact de la plaque avec la surcouche et avant chaque étape d'enlèvement de la surcouche, le laminé respectif plaque-élément est étuvé à une température allant jusqu'à 45 °C et pendant une durée suffisante pour permettre à la substance à analyser de réagir avec la composition interactive respective.

5. Nécessaire de diagnostic pour la détermination d'une ou plusieurs substances à analyser, comprenant: une plaque adaptée pour une migration électro-induite d'une substance à analyser, et au moins un élément pour analyse à sec comprenant un liant insoluble dans l'eau dans lequel est dispersée une composition interactive capable de réagir avec la substance à analyser pour former seulement dans l'élément une espèce non détectable non-diffusible.

6. L'invention selon l'une des revendications 1 à 5, dans laquelle les substances à analyser sont des protéines ou des acides nucléiques.

7. L'invention selon l'une des revendications 1 à 6, dans laquelle les substances à analyser sont des isoenzymes.

8. L'invention selon l'une des revendication 1 à 7, dans laquelle les substances à analyser sont les isoenzymes de la créatine kinase, de la phosphatase alcaline ou de la lactate déshydrogénase.

9. L'invention selon des revendications 1 à 8, dans laquelle chaque élément d'analyse est pratiquement transparent et chaque composition interactive réagit pour former un chromogène non-diffusible.

10. L'invention selon l'une des revendications 1 à 9, dans laquelle chaque élément d'analyse à sec comprend un support avec au moins une couche contenant le liant et la composition interactive.

11. L'invention selon l'une des revendications 1 à 10, dans laquelle le liant est la gélatine tannée.